# EUROPEAN PATENT SPECIFICATION

(11) **EP 4 164 592 B1**
(45) Date of publication and mention of the grant of the patent: **07.05.2025**
(21) Application number: 20732831.1
(22) Date of filing: 10.06.2020
(51) Int. Cl.: A61K 9/00, A61K 9/50, A61K 31/00, A61K 47/10, A61K 47/36, A61P 3/00, A61P 9/00, A61P 11/00

(54) **PHARMACEUTICAL COMPOSITIONS CONTAINING ENTEROKINE-RELEASING SUBSTANCES IN MULTIPLE DOSAGE FORMS IN COMBINATION WITH GELLING AGENTS**
PHARMAZEUTISCHE ZUSAMMENSETZUNGEN, DIE ENTEROKINFREISETZENDE SUBSTANZEN IN MEHRFACHDOSISFORMEN IN KOMBINATION MIT GELIERMITTELN ENTHALTEN
COMPOSITIONS PHARMACEUTIQUES CONTENANT DES SUBSTANCES LIBÉRANT DE L'ENTÉROCINE SOUS FORME DE DOSES MULTIPLES EN COMBINAISON AVEC DES GÉLIFIANTS

(43) Date of publication of application: 19.04.2023
(73) Proprietor: Aphaia IP AG, 6300 Zug (CH)
(72) Inventor: Bolz, Steffen-Sebastian, 82211 Herrsching (DE); Deusch, Kai, 82057 Icking (DE)
(74) Representative: Habermann, Hruschka & Schnabel
(86) International application number: PCT/EP2020/066149
(87) International publication number: WO 2021/249641

(56) References cited:
- WO-A1-2014/032741
- WO-A1-2016/096931
- WO-A1-2019/170840
- US-A1- 2006 134 210
- US-A1- 2014 017 363
- CHOE S Y ET AL: "Novel method to assess gastric emptying in humans: the Pellet Gastric Emptying Test", EUROPEAN JOURNAL OF PHARMACEUTICAL SCIENCES, ELSEVIER AMSTERDAM, NL, vol. 14, no. 4, 1 December 2001 (2001-12-01), pages 347 - 353, XP002265976, ISSN: 0928-0987, DOI: 10.1016/S0928-0987(01)00196-8
- FANG LIU: "Patient-Centered Pharmaceutical Design to Improve Acceptability of Medicines: Similarities and Differences in Paediatric and Geriatric Populations", DRUGS, vol. 74, no. 16, 2 October 2014 (2014-10-02), NZ, pages 1871 - 1889, XP093156412, ISSN: 0012-6667, Retrieved from the Internet <URL:https://www.ncbi.nlm.nih.gov/pmc/articles/PMC4210646/pdf/40265_2014_Article_297.pdf> DOI: 10.1007/s40265-014-0297-2
- TIRPUDE RAKESH N ET AL: "Drug Multiparticulate Production and Coating Technology - A Review", RESEARCH JOURNAL OF PHARMACY AND TECHNOLOGY, A & V PUBLICATIONS, INDIA, vol. 4, no. 1, 1 January 2011 (2011-01-01), pages 1 - 18, XP009172066, ISSN: 0974-3618

## Description

The present invention relates to a pharmaceutical composition as defined in claim 1 and a pharmaceutical article as defined in claim 18.

The invention also relates to medical uses of the pharmaceutical composition and the pharmaceutical article as defined in claim 24.

It has early been recognised that delivery of a nutritional substance to the ileum through use of an enteric dosage form of the nutritional substance leads to satiety of a mammalian subject (US 5753253 A). More recently, specific delivery of a nutritional substance, in particular glucose, to the ileum of a subject, in particular dosage forms of a nutritional substance like glucose, which dosage forms release at least 50 % of the administered substance in the ileum of the subject, was suggested for treatment of metabolic diseases like type 2 diabetes mellitus, metabolic syndrome, insulin resistance, obesity, non-alcoholic fatty liver disease (NAFLD), non-alcoholic steatohepatosis (NASH) and other conditions (WO 2010/027498 A2, WO 2012/118712 A2, US 2014/0294951 A1).

Co-pending International Patent Application PCT/EP2019/084530 relates to pharmaceutical oral dosage forms comprising a core and a pH-sensitive enteric coating, wherein the core comprises at least one compound stimulating enteroendocrine cells to release at least one enterokine, and at least one disintegrant providing a burst release of the ingredients of the core when the coating is substantially degraded and/or dissolved, and wherein the coating comprises a pH sensitive polymer being selected such that the coating substantially dissolves and/or is substantially degraded in the jejunum of a subject. Co-pending International Patent Application PCT/EP2019/084531 relates to combinations of at least two pharmaceutical oral dosage forms for use in the prevention and/or treatment of a condition, disorder or disease selected from the group consisting of insulin resistance, type 2 diabetes, non-alcoholic fatty liver disease, non-alcoholic steatohepatosis, microvascular dysfunction, metabolic syndrome, obesity, osteoporosis, neurodegenerative diseases, cardiovascular diseases, malabsorption conditions and conditions of impaired gastro-intestinal function in a subject wherein the combination comprises at least a first and a second dosage form each comprising a core and a pH-sensitive enteric coating, wherein each core comprises at least one compound stimulating enteroendocrine cells to release at least one enterokine, and at least one disintegrant providing a burst release of the ingredients of the core when the coating is substantially degraded and/or dissolved, and wherein each coating comprises a pH sensitive polymer being selected such that the coating substantially dissolves and/or is substantially degraded in a selected part of the small intestine of a subject, wherein said first and second dosage form are formulated such that said first dosage form releases the at least one compound stimulating enteroendocrine cells to release at least one enterokine in a different part of the small intestine of the subject than the second dosage form and/or the first and second dosage form are formulated such that said first dosage form show a travelling time through the small intestine of the subject being different from the travelling time of the second dosage form through the small intestine of the subject;
wherein the at least one compound stimulating enteroendocrine cells to release at least one enterokine of said first and second dosage form can be the same or different.

WO 2016/096931 A1 and WO 2019/170840 A1 disclose microencapsulates comprising a liquid core encapsulated within a polymerized denatured protein shell wherein the core comprises a GLP-1 release stimulating agent.

WO 2014/032741 A1 relates to gastric resistant pharmaceutical or nutraceutical compositions with resistance against the influence of ethanol.

US 2006/0134210 A1 discloses solid rapidly gelling pharmaceutical dosage forms of proton pump inhibitors.

Choe et al. (2002) Eur. J. Pharm. Sci. 14, 347-353, disclose a novel test method for assessing gastric emptying in humans.

Liu et al. (3014) Drugs 74, 1871-1889 discuss patient-centered pharmaceutical designs for improving acceptability of medicines in paediatric and geriatric patients.

The technical problem underlying the present invention is to provide improved preventive and therapeutic measures conditions associated with disturbed enterokine release by enteroendocrine cells and/or amenable to increased enterokine release by enteroendocrine cells.

The solution to the above technical problem is provided by the embodiments of the present invention as defined in the claims.

In particular, the present invention provides a pharmaceutical composition comprising multiple dosage forms each comprising a core and an enteric coating, wherein the core comprises at least one nutrient compound selected from the group consisting of carbohydrates, fatty acids, bile acids, peptides, amino acids, alcohol amides, and anthocyanins stimulating enteroendocrine cells to release at least one enterokine, wherein the size of the dosage forms, with respect to the largest dimension of the dosage forms, is below 3 mm providing for entry of the dosage forms into the intestine of a subject independent of gastric emptying mechanisms, wherein the enteric coating comprises a pH sensitive polymer which substantially degrades and/or dissolves at a pH value being selected such that the coating substantially dissolves and/or is substantially degraded in the terminal jejunum of a subject such that the core is released into the terminal jejunum of the subject,and wherein the composition further comprises a gelling composition comprising one or more gelling agents wherein the gelling composition and the multiple dosage forms form a heterogenous mixture.

A "dosage form" according to the invention is an entity which represents in itself a pharmaceutical formulation typically, and most preferably suitable for oral administration to a subject, preferably a human subject. The multiple dosage forms present in the pharmaceutical composition of the invention are dimensioned, i.e. have size with respect to their largest dimension, such that, when the pharmaceutical composition of the invention is administered orally to the patient, the multiple dosage forms enter the intestine independent of gastric emptying mechanisms, in particular peristaltic waves, contractions of the Antrum, and shrinkage of the size of the stomach. This means that the multiple dosage forms behave, as regards their transition from the stomach into the intestine, in particular the most proximal part of the small intestine, namely, the duodenum, as a fluid.

The skilled person understands that the parameters of the multiple dosage forms, in particular the size of the multiple dosage forms, their number in the inventive composition, in particular in a unit dose of the composition according to the invention, are interdependent and usually depend on further parameters, such as in particular the specific type and amount of the active pharmacological ingredient(s) (API), specifically the at least one compound stimulating enteroendocrine cells to release at least one enterokine.

That being said, the size of each of the dosage forms present in the composition of the invention, with respect to the largest dimension of the dosage forms, is below 3 mm, preferably from about 0.6 mm to about 3.0 mm, more preferably from about 0.6 mm to about 2.6 mm, even more preferred from about 0.6 mm to about 1.7 mm, and most preferred from about 0.8 mm to about 1.2 mm. The multiple dosage forms contained in the composition according to the invention are typically present in form of pellets, beads or granules, with beads, particularly round-shaped beads, being preferred forms.

The composition of the invention contains a multitude of said dosage forms. The term "multiple dosage forms" typically means that the composition contains at least about1000, more preferably at least about 2000, more preferably at least about 3000, particular preferred at least about 5000, even more preferred at least about 10000 dosage forms. Preferred ranges are from about 1000, about 2000, about 3000, about 5000, or about 10000 to about 20000, about 30000, about 40000 or about 50000 dosage forms in the composition of the invention, preferably per unit dose of the composition according to the invention. Especially preferred ranges are about 10000 to about 40000 dosage forms, and most preferred compositions of the invention contain about 20000 to about 30000 dosages forms. In particular with respect to such embodiments, the size of the dosage forms preferably ranges, according to the above definition, from about 0,6 mm to 1,7 mm, and most preferred from about 0,8 mm to about 1,2 mm.

According to particularly preferred embodiments of the invention, the size and number of the multiple dosage forms contained in the inventive pharmaceutical composition is controlled such that the surface area of the multiple dosage forms in the composition, especially in a unit dose of the composition, is selected such that it covers at least 15 %, more preferably at least 20 %, particularly preferred at least about 25 % of the target area where the at least one active compound as defined herein interacts with the enteroendocrine cells so as to induce a release of the enterokine. In this context, it is to be understood that said target area is not necessarily confined, and in certain embodiments of the invention cases preferably not confined, to the area where the core of the multiple dosage forms releases the active compound. The target area of the active compound is typically dependent on the type of enteroendocrine cells to be triggered to release the enterokine(s). As a preferred example, L cells which are preferably induced by the active compound(s) in the core of the multiple dosage forms are, as further outlined below, present in humans in the small intestine from duodenum to ileum, but their enterokine release capacity, specifically GLP-1 release capacity, particularly due to the increased density and differentiation of the enteroendocrine cells, increases from proximal to distal small intestine, and is highest in the terminal ileum According to the invention, especially for triggering L cells to release GLP-1 and/or PYY, preferably GLP-1, the enteric coating of the multiple dosage forms is selected such that the at least one active compound is released from the core of the multiple dosage forms in the terminal jejunum of a subject, and the surface area of the multiple dosage forms of the composition of the invention, preferably a unit dose of the composition, is selected such that said surface area makes up between from about 10 % to about 50 %, preferably from about 15 about 35 %, more preferred about 20 to about 30 % of the targeted part of the intestine for the active compound where the targeted cells, preferably L cells, have their highest concentration and/or enterokine release capacity, which is for L cells the ileum, in particular the terminal ileum. In this contest, the term "surface area of the target part of the intestine" is approximated as taken the intestine as a tube having a mean diameter of about 0.25 cm and a length of the terminal ileum of about 60 cm.

The pharmaceutical composition of the invention further contains, as essential ingredient, a gelling composition comprising at least one gelling agent. Gelling agents, also known as thickeners, are gel-forming agents when dissolved in a liquid phase as a colloidal mixture forms a weakly cohesive internal structure. They are typically organic hydrocolloids or hydrophilic inorganic substances. Typical examples include tragacanth, pectin, starch, carbomers, polysaccharides, gelatin, cellulose derivatives, polyvinyl alcohol clays, polyethylene glycols and others (for a review, see, e.g. Kar et al. "Current Developments in Excipient Science, Section 2.2.2.5 "Gelling Agents" in: R. Tekade (ed.) Basic Fundamentals in Drug Delivery, Academic Press, Cambridge, MA, USA, 2019). The term "at least one gelling agent" as used herein also includes compositions of one or more gelling agents, optionally in combination with other components.

Preferred gelling agents, either for use alone or for use in gelling compositions containing combinations of such gelling agents, include, but are not limited to, modified celluloses such as preferably substituted methylcelluloses, such as hydroxypropylmethylcelluloses (HPMCs), polysaccharides such as preferably alginates, e.g. sodium alginate, and Xanthan gum, and polyethylene glycols (PEGs), preferably PEGs having a mean molecular weight of from about 10000 Da to about 30000 Da, more preferably 15000 Da to about 25000 Da, most preferred about 20000 Da. In preferred embodiments of the invention, the composition contains more than one gelling agents in order to fine tune the properties of the composition, in particular with respect to a specific design of the viscosity properties so as to greatly improve swallowability of the composition in use, when containing or when reconstituted, respectively, with a, preferably pharmaceutically acceptable, liquid medium, e.g. water or an aqueous solution. Preferred combinations of gelling agents, typically in form of a gelling composition, include 2, 3 or 4 gelling agents. Particularly preferred combinations of gelling agents for use in the inventive composition are selected from combinations of modified celluloses, polysaccharides and PEGs, more preferably one or more of the above-mentioned preferred examples.

The pharmaceutical composition of the invention can be provided in various forms. In one preferred embodiment, the pharmaceutical composition according to the invention contains a liquid medium so that the multiple dosage forms and the gelling composition comprising one or more gelling agent are present in a liquid medium, preferably water or an aqueous solution, more preferably a buffered aqueous solution. According to the invention, the gelling composition and the multiple dosage forms form a heterogeneous mixture. For use of the pharmaceutical composition, a liquid medium, as described above and further disclosed herein, is preferably added to the composition. For a unit dose of the composition, a volume of the liquid is selected typically based on the number of the multiple dosage and/or their size. For preferred values of the multiple dosage forms, the dry embodiment of the pharmaceutical composition is typically admixed with about 5 ml to about 40 ml, preferably about 10 to about 35 ml, most preferred about 15 to 30 ml of the liquid medium.

In order to provide stabilization of the inventive pharmaceutical composition upon oral administration, in which form it typically comprises a suitable liquid medium, in particular for stabilization of the gel or gel-like formulation of the inventive composition in the milieu of mouth, throat and/or oesophagus, the composition of the invention preferably comprises one or more pH modifiers, such as preferably citric acid, a salt of citric acid such as tri-potassium citrate monohydrate, adipic acid, sodium hydrogen carbonate, tartaric acid and combinations thereof. In further preferred embodiments of the invention, the one or more pH modifiers can also be included in the gelling composition as described above.

An "enteroendocrine cell" (EEC) as used in the present invention, is a cell present in a subject's, preferably, human subject's, intestine, in particular in the small intestine's mucosa, and secretes one or more enterokines upon receiving an appropriate trigger by a nutritional component. There are several types of enteroendocrine cells which can be addressed by the active compound or compounds released by the pharmaceutical composition of the invention (for a review of enteroendorine cells of the lower gastrointestinal tract, see, for example, Gunawardene et al. (2011) Int. J. Exp. Pathol. 92, 219-231, and Latorre et al. (2017) Neurogastrol. Motil 28 (5), 620-630). In particular, preferred enteroendocrine cells in the context of the invention are I cells, K cells and L cells. with L cells being most preferred.

According to the invention, an "enterokine‴ is a hormone secreted by EECs in the gastro-intestinal system. Preferred enterokines are the incretins, i.e. hormones regulating the blood sugar level upon intake of nutrition, preferably GLP-1, GLP-2 GIP and PYY, more preferably GLP-1 and PYY. Other preferred enterokines secreted by the EEC(s) through release of the active compound(s) in the pharmaceutical composition are CCK and neurotensin.

I cells are predominantly present in the proximal small intestine, in particular in the lower duodenum and in the jejunum and secrete cholecystokinin (CCK) upon sensing of nutrients such as amino acids and fatty acids. CCK effects the release of bile acids from the gall bladder into the small intestine, but also promotes the release of digestive secrete from the pancreas.

K cells are found in the complete small intestine and release GIP (glucose-dependent insulinotropic peptide; also known as gastric inhibitory peptide) leading to inhibition of gastric motility and enhancement of insulin production and secretion.

L cells are present throughout the small intestine, i.e. duodenum, jejunum, and ileum, and release GLP-1 (glucagon-like peptide 1), GLP-2 (glucagon-like peptide 2) and PYY (peptide YY) in response to various nutrients. The concentration of L cells raises from duodenum to jejunum to ileum, and the GLP-1 release from L cells shows a gradient sloping from proximal to distal small intestine with highest GLP-1 release capacity in the terminal ileum. The gradient is not only a function of increasing number of L cells, but also a function of their maturation and differentiation state as well as production rate of GLP-1, which also increase from proximal to distal ileum. In particular, GLP-1 is released from L cells in the crypts and on the villi of the mucosa. L cells mature from crypt to villi, and GLP-1 release capacity is highest when the L cells reach the top of the villi. L cells further secrete PYY (peptide YY) which is predominantly released in the terminal ileum. GLP-1 enhances nutrient-stimulated insulin secretion and inhibits glucagon secretion, gastric emptying and feeding. GLP-1 also has proliferative, neogenic and antiapoptotic effects on pancreatic β-cells. As an intestinal trophic peptide, GLP-2 stimulates cell proliferation and inhibits apoptosis in the intestinal crypt compartment, it also regulates intestinal glucose transport, food intake and gastric acid secretion and emptying. Furthermore, GLP-2 improves intestinal barrier function. PYY inhibits gastric motility and increases water and electrolyte absorption in the colon. PYY also suppresses pancreatic secretion and has been shown to reduce appetite. PYY slows gastric emptying; whereby it increases efficiency of digestion and nutrient absorption after a meal.

EECs, in particular L cells, are triggered to release enterokines such as GLP-1 and PYY through a variety of mechanisms being affected by the one or more active compound(s) in the pharmaceutical composition of the invention.

L cells release GLP-1 and PYY (and also GLP-2; note that GLP-1 and GLP-2 are derived from a common mRNA so that these hormones are essentially co-released; cf., for example, the review of Baggio and Drucker (2004) Best Practice & Research Clinical Endocrinology & Metabolism 18 (4), 531-554) in response to various mechanisms triggered by compounds such as nutrients. One mechanism includes intake of a carbohydrate such as glucose through glucose transporters GLUT2 and/or SGLT1. Other mechanisms rely on the binding to specialized G protein-coupled receptors such as taste receptors, fatty acid receptors, bile acid receptors, peptide receptors and amino acid receptors.

These signals, glucose transport and binding to G protein-coupled receptor, are typically transmitted in the cells by one or more of three mechanisms and lead ultimately to the release of the enterokine, in the case of L cells GLP-1 and PYY: transmembrane calcium influx, intracellular calcium release and/or intracellular cAMP increase.

Accordingly, the active compound(s) triggering the release of an enterokine through an EEC are selected from nutrients selected from carbohydrates, fatty acids, bile acids, peptides (including oligopeptides, polypeptides and proteins), amino acids, alcohol amides and anthocyanins. Preferred fatty acids are fatty acids having 2 to 6 carbon atoms. Ethanolamides such as oleoylethanolamide, anandamide (N-arachidonoylethenolamide, AEA), palmitoylethanolamide, steaorylethanolamide, and derivatives of anandaminde such as prostamides, can also preferably be used as alcohol amide compounds in the inventive oral dosage forms. A particularly preferred ethanolamide is oleoylethanolamide (OEA). A preferred example of a peptidic active compound is the protein bovine serum albumin (BSA). Carbohydrates are preferably selected from glucose and sucralose, with glucose being most preferred. In a particular preferred embodiment of the invention, the multiple dosage forms in the pharmaceutical composition contain about 1 % (w/w) to about 99 % (w/w), such as 60 to 70 % (w/w) of active compound triggering the EECs, preferably a carbohydrate, most preferred glucose (dextrose). Particularly preferred glucose contents in the core of the multiple dosage forms are in the range of form about 5 % (w/w) to about 95 % (w/w), preferably from about 25 % (w/w) to about 75 % (w/w), more preferably from about 40 % (w/w) to about 70 % (w/w).

In a preferred embodiment, the active compound triggering the release of an enterokine through an EEC is an anthocyanin, more preferably one or more anthocyanins from *Vaccinium myrtilloides* Michx. A particularly preferred anthocyanin to be included in the core of the pharmaceutical oral dosage forms of the invention is delphinidin 3-rutinoside.

In a preferred embodiment of the invention, one or more of the above active compounds triggering enterokine release by EECs are combined in preferably synergistic combinations.

Preferred combinations are combinations of a carbohydrate, preferably glucose, with sucralose and/or one or more fatty acids having 2 to 6 carbon atoms and/or oleic acid and/or one or more bile acids and/or one or more alcohol amides, preferably one or more ethanolamides, more preferably oleoylethanolamide, and/or one or more peptides (including oligopeptides, polypeptides and proteins, such as preferably BSA) and/or one or more amino acids and/or one or more anthocyanins (preferably delphinidin 3-rutinoside).

Further preferred combinations are combinations of another carbohydrate, preferably sucralose, with glucose and/or one or more fatty acids having 2 to 6 carbon atoms and/or oleic acid and/or one or more bile acids and/or one or more alcohol amides, preferably one or more ethanolamides, more preferably oleoylethanolamide, and/or one or more peptides (including oligopeptides, polypeptides and proteins) such as preferably BSA, and/or, one or more amino acids and/or one or more anthocyanins (such as preferably delphinidin 3-rutinoside).

Other preferred combinations are combinations of a fatty acid having 2 to 6 carbon atoms with one or more carbohydrates, preferably glucose and/or sucralose, and/or oleic acid and/or one or more bile acids and/or one or more alcohol amides, preferably one or more ethanolamides, more preferably oleoylethamolamide, and/or one or more peptides (including oligopeptides, polypeptides and proteins), preferably BSA) and/or one or more amino acids and/or one or more anthocyanins such as preferably delphinidin 3-rutinoside.

According to a further preferred embodiment, the present invention provides combinations of oleic acid with one or more fatty acids having 2 to 6 carbon atoms and/or one or more carbohydrates, preferably glucose and/or sucralose, and/or one or more bile acids and/or one or more alcohol amides, preferably one or more ethanolamides, more preferably oleoylethanolamide and/or one or more peptides (including oligopeptides, polypeptides and proteins, such as preferably BSA) and/or one or more amino acids and/or one or more anthocyanins such as preferably delphinidin 3-rutinoside.

Further preferred combinations of active compounds are combinations of one or more bile acids with one or more carbohydrates, preferably sucralose and/or glucose, and/or one or more fatty acids having 2 to 6 carbon atoms and/or oleic acid and/or one or more alcohol amides, preferably one or more ethanolamide, more preferably oleoylethanolamide, and/or one or more peptides (including oligopeptides, polypeptides and proteins, preferably BSA, and/or one or more amino acids and/or one or more anthocyanins such as preferably delphinidin 3-rutinoside.

Still further preferred combinations of active compounds are combinations of one or more alcohol amides, preferably one or more ethanolamides, more preferably oleoylethanolamide, with one or more carbohydrates, preferably sucralose and/or glucose, and/or one or more fatty acids having 2 to 6 carbon atoms and/or oleic acid and/or one or more bile acids and/or one or more peptides (including oligopeptides, polypeptides and proteins) preferably BSA, and/or one or more amino acids and/or one or more anthocyanins such as preferably delphinidin 3-rutinoside.

Other preferred combinations of active compounds are combinations of one or more peptides (including oligopeptides, polypeptides and proteins), preferably BSA, with one or more carbohydrates, preferably sucralose and/or glucose, and/or one or more fatty acids having 2 to 6 carbon atoms and/or oleic acid and/or one or more bile acids and/or one or more alcohol amide, preferably one or more ethanolamide, more preferably oleoylethanolamide, and/or one or more amino acids and/or one or more anthocyanins such as preferably delphinidin 3-rutinoside.

In other preferred embodiments of the invention, the core of the multiple dosage forms contains a combination of one or more amino acids with one or more carbohydrates, preferably sucralose and/or glucose, and/or one or more fatty acids having 2 to 6 carbon atoms and/or oleic acid and/or one or more bile acids and/or one or more ethanolamides, more preferably oleoylethanolamide, and/or one or more peptides (including oligopeptides, polypeptides and proteins); preferably BSA, and/or one or more anthocyanins such as preferably delphinidin 3-rutinoside.

In still further preferred combinations of active compounds in the core part of the multiple dosage forms used in the invention, one or more anthocyanins, preferably delphinidin 3, is/are combined with one or more carbohydrates, preferably sucralose and/or glucose, and/or one or more fatty acids having 2 to 6 carbon atoms and/or oleic acid and/or one or more bile acids and/or one or more alcohol amides, preferably one or more ethanolamides, more preferably oleoylethanolamide, and/or one or more peptides (including oligopeptides, polypeptides and proteins), preferably BSA, and/or one or more amino acids.

The active compound(s) in the core part of the multiple dosage forms are preferably further combined with active ingredients having various functions.

Preferred examples of additional active ingredients are EEC maturation agents. As exemplified before with L cells such maturation agents typically enhance the release capacity of the EECs, such as L cells, for the relevant enterokine, in the case of L cells GLP-1 and/or PYY and/or GLP-2. Preferred EEC maturation agents, in particular L cell maturation agents, include human milk oligosaccharides (HMO) and inhibitors of NOTCH signalling such as γ-secretase inhibitors, preferably dibenzazepine. Various HMOs are commercially available (Jennewein Biotechnologie GmbH, Rheinbreitbach, Germany) and preferred HMOs in the context of the invention include, but are not limited t, e.g. 2'-fucosyllactose, 3-fucosyllactose, 6'-sialyllactose and lacto-N-neotetraose as well as mixtures thereof.

The pharmaceutical composition of the invention preferably contains multiple oral dosage forms designed to burst release the active ingredient(s) at a selected targeted area of the small intestine of a subject, namely, the terminal (= distal) jejunum) of the, preferably human, subject. For providing a burst release of the ingredients of the core of the multiple dosage forms, the core of the multiple dosage forms contains at least one disintegrant. Disintegrants for use in the present invention are generally known in the art as rapidly expanding and dissolving when coming into contact with the targeted environment, typically, an aqueous environment as in the present invention, namely, the small intestine of a subject, preferably a human. Disintegrants lead to rapid breakdown of the core of the pharmaceutical oral dosage form of the invention when the core comes into contact of the aqueous medium present in the subject's small intestine. Preferably, the disintegrant in the multiple dosage forms of the composition of the invention is selected such that more than 70 % of the core is released within two minutes or less or more than 85 % of the core is released within 5 minutes or less, following contact with water or an aqueous medium like the small intestine of a human subject. Preferred disintegrants in the context of the invention are crosslinked polyvinylpyrrolidones, crosslinked carboxymethyl celluloses and modified starchs. Particularly preferred crosslinked polyvinylpyrrolidones for use in the invention include Polyplasdones, in particular Polyplasdone XL, Polyplasdone XL-10 and Polyplasdone INF-10 (commercially available from Ashland Inc., Covington, KY, USA). Other useful disintegrants include, but are not limited to, polyvinylpolypyrrolidone, croscarmellose and carboxymethylcellulose (preferably, the sodium salt thereof).

A burst release of the core's ingredients, in particular the active compound(s) such as glucose, establishes a steep gradient between intracellular and extracellular levels of the respective active compound(s), preferably glucose, sucralose, ethanolamides, BSA and/or an anthocyanine such as delphinidin 3-rutinoside, in the targeted EEC, preferably L cells, at the site of release of the core ingredients.

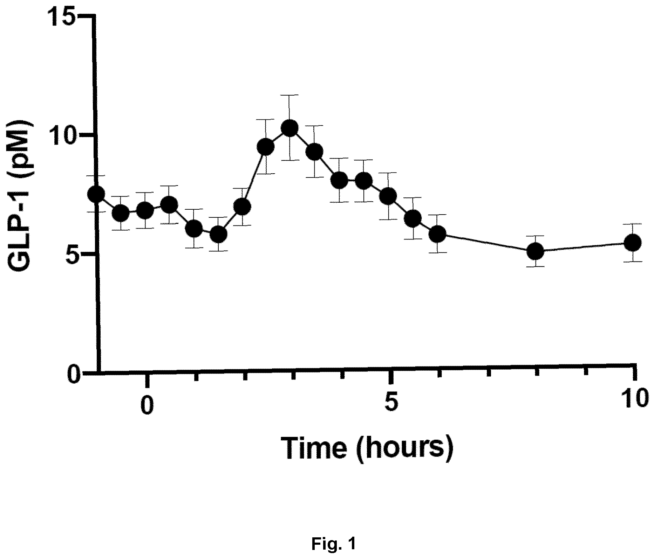

Compared to previous technologies, in particular WO 2010/027498 A2, WO 2012/118712 A2 and US 2014/0294951 A1, the pharmaceutical composition of the invention shows a combination of highly improved properties: on the one hand side, the pharmaceutical composition of the invention provides for an improved release of enterokine release, in particular GLP-1 and/or PYY release, in patients, especially highly reproducible strong enterokine release combined with very low intra-patient and inter-patient variability, since every dose of the composition of the invention provides for a single peak of enterokine, in particular GLP-1, release at the selected time point after oral administration (see, in particular, Fig. 1 of the present application in comparison to the results obtained with prior art compositions such as, e.g. in US 2014/0294951 A1; cf. Fig. 6a to 6f thereof). On the other hand, due to the combination of the multiple dosage forms with the gelling agent(s), preferably present in a gelling composition, the pharmaceutical composition described herein is a very safe formulation of the comparatively small multiple dosage forms, which formulation, in particular when reconstituted with a liquid medium such as water or an aqueous solution, provides a suspension of the multiple dosage forms in a gel or gel-like formulation as disclosed herein, which can be swallowed substantially more easily by the subject (as compared, for example, to comparatively large capsules or tablets of 10 g of API (glucose) disclosed in WO 2010/027498 A2 and WO 2012/118712 A2) in need thereof contributing to improved compliance. Furthermore, due to the presence of the at least one gelling agent, it is prevented that the multiple dosage forms, small in size, may be aspired by the subject. This is of high importance both for safety and patient compliance, since oral administration of large tablets or capsules containing, e.g. 7 to 10 g glucose, as taught in WO 2010/027498 A2 and WO 2012/118712 A2 in order to obtain any effect, is particularly difficult for children, such as preferably pediatric patients having an age of up to about 6 years of age, and elderly patients, preferably elder patients in the age ranges outlined below. It is most important for the subjects preferably addressed by the present composition (in particular, subjects suffering from obesity, insulin resistance, type 2 diabetes mellitus (T2DM), metabolic syndrome, but also severe forms of certain viral infections such as COVID-19) are typically elderly subjects such as subjects being of an age of at least about 50, more preferably at least about 55, even more preferred at least about 60, which in general show a statistically high incidence of swallowing problems, up to severe dysphagia and even achalasia, and the specific formulation of the present can be easily and safely taken up be such subjects, even in case of dysphagia, which will provide for a much higher compliance rate and consequently highly improved positive effects in the course of treatment of patients compared to prior art technologies.

Moreover, and of particular relevance, the inventive combination of multiple dosage forms and gelling agent(s) (present in a gelling composition), and optionally further agents such as, in particular pH modifying agents, shows an improved viscosity and flow properties on mucosal surface to optimize ingestion in particular in subjects of the elderly and pediatric populations, whereby preferred age ranges of subjects of these populations are given above. In order to further improve the flow properties of the pharmaceutical composition, in particular when containing or when reconstituted with a liquid medium as disclosed above, respectively, one or more viscosity modifiers (in particular other than gelling agents) and/or one or more flow aids (in particular other than gelling agents) can be included into the pharmaceutical compositions of the invention. Preferred examples of substances useful as viscosity modifiers and/or flow aids are lubricants such as preferably polyalcohols, more preferably glycerol, colloidal silicon dioxides and vegetable oils, more preferably one or more vegetable oils selected from olive oil, sunflower seed oil, almond oil and sesame oil.

According to the invention, the multiple dosage forms release the active compound(s) in the terminal jejunum, of a subject, preferably a human subject, through a specific design of the enteric coating, typically a pH sensitive coating. The coating substantially degrades and/or dissolves in the terminal jejunum, such that the core is released into the terminal jejunum, of the subject, by specific selection of the enteric coating which is preferably chosen from pH sensitive polymers substantially degrading and/or dissolving at a pH value of about 5.5 to about 7.5, preferably about 7.2 to about 7.3. Such pH sensitive polymers are preferably selected from hydroxypropylmethyl celluloses (also called hereinafter "hypromelloses") and anionic copolymers of methacrylic acid and methacrylmethacrylate. Most preferably, the pH sensitive enteric coating containing or being made of hydroxypropylmethyl cellulose is hydroxypropylmethyl cellulose acetate succinate. A highly preferred commercially available product of this kind is AQOAT^{®}, particularly preferred AQOAT^{®}-HF (Shin-Etsu Chemical Co., Chiyoda, Japan). In other preferred embodiments of the type of anionic copolymers of methacrylic acid and methcrylmethacrylate various forms of Eudragit^{®} polymers may also be used. Eudragit^{®} is commercially available from Evonik Healthcare & Nutrition GmbH, Essen, Germany. In preferred embodiment, Eudragit^{®} FS30D and/or Eudragit^{®} L30D is/are used as the pH sensitive polymer(s) of the coating, or at least a part thereof.

In further preferred embodiments of the invention, different coatings can be applied in combination. According to one embodiment, the coating comprises or is made of a combination of a hydroxypropylmethyl cellulose and an anionic copolymer of methacrylic acid and methacrylmethacrylate. Preferably, a combination of coatings is applied such that typically a sub-coating of one pH sensitive polymer is applied as a first layer and a coating of a second pH sensitive polymer is applied on the sub-coating as a second layer. For example, the pH sensitive coating can comprise a sub-coating of or comprising, respectively, a hydroxypropylmethyl cellulose as a first layer, and a second coating comprising or being made of an anionic copolymer of methacrylic acid and methacrylmethacrylate provided as a second layer on the sub-coating. In a further preferred embodiment, the coating of the pharmaceutical oral dosage form of the invention comprises a coating comprising a first layer (sub-coating) comprising or being made of an anionic polymer of methacrylic acid and methacrylmethacrylate such as an Eudragit^{®}, more preferably Eudragit^{®} FS30D, and a second layer comprising or being made of a hydroxypropylmethyl cellulose such as AQOAT^{®}, more preferably AQOAT^{®}-HF. More preferably, the anionic copolymer of methacrylic acid and methacrylmethacrylate, e.g. an Eudragit^{®}, preferably Eurdragit^{®} FS30D, is present in less amount than the hydroxypropylmethyl cellulose such as AQOAT^{®}, more preferably AQOAT^{®}-HF. In other words, the thickness of the first layer of this type of combination is lower than the thickness of the second layer in this combination. More specifically, the ratio of amount or thickness, respectively, between first layer and second layer typically ranges from about 1:10 to about 1:50, particularly preferred from about 1:20 to about 1:30.

For further improving the enterokine release exerted by the inventive composition, it is preferred that the multiple dosage forms, in particular their core, further contain enteroendocrine release improvement agents, i.e. a substance enhancing enterokine release by the enteroendocrine cells effected by the compound stimulating enteroendocrine cells to release at least one enterokine, particularly preferred at least one substance enhancing release of GKLP-1 and/or PYY by L cells. For preferred embodiments of the invention where L cells are targeted to release GLP-1 such agents are preferably selected from inhibitors of GLP-1 degradation such as DPP-4 inhibitors, substances enhancing L cell maturation (e.g. human milk oligosaccharides, inhibitors of NOTCH signalling such as γ-secretase inhibitors, preferably dibenzazepine), stimulating agents and combinations thereof. A preferred stimulating agent for use in the present invention is caffeine. One example of a preferred combination of active compound and stimulating agent is glucose and caffeine, preferably in a weight ratio of glucose to caffeine of from about 5:1 to about 50:1, preferably about 8 to 1 to about 40:1. In other embodiments, preferred combinations of glucose and caffeine in the core of the multiple oral dosages forms are about 60 to about 70 % (w/w) glucose and about 2 to about 4 % (w/w) caffeine, most preferred about 67 % (w/w) glucose and about 3.2 % (w/w) caffeine, based on the total weight of the core.

Caffeine and other known substances can also be included for controlling and monitoring the appropriate release and also the spreading of the ingredients of the core of the multiple oral dosage forms contained in the composition of the invention in the intestinal tract of the subject, preferably a human. The release of the tracer substance from the multiple dosage forms of the pharmaceutical composition in the small intestine can be conveniently monitored by analytical methods generally known in the art. In the case of caffeine, blood samples are taken from the subject before and at suitable time intervals after oral administration of the oral dosage form. After centrifugation of the blood sample, the caffeine content in the serum fraction of the sample is measured by ELISA testing using commercially available test kits (e.g. Caffeine ELISA Kit, BioVision Inc., Milpitas, CA, USA) according to the manufacturer's instructions.

The pharmaceutical composition forms of the invention are preferably fine-tuned in various further ways so as to further improve targeted release and triggering of EECs in the small intestine of a subject, preferably a human subject. Moreover, different multiple dosage forms having a variety of release patterns in the subject's small intestine can be combined in the composition of the invention so as to synergistically act on the subject's EECs and their enterokrine output.

As outlined above, the present invention is directed to specific pharmaceutical compositions comprising preferable multiple dosage forms as defined in claim 1 which are small in dimension, i.e. below 3 mm in the largest dimension, preferably about 0.6 mm to about 1.7 mm in the largest dimension, particularly preferred from about 0.8 mm to about 1.2 mm in the largest dimension. As already outlined above, such small dosage forms may conveniently take the form of beads, granules or pellets. As has been explained in detail above, the small dosage forms of the invention have the benefit of behaving like a fluid in a subject's stomach causing a fast and constant entry of the multiple oral dosage forms into the intestinal tract, and therefore to evenly transport it to the targeted area, namely the terminal jejunum.

In addition to the above components the pharmaceutical composition of the invention may contain further ingredients typically present in oral dosage forms such as tablets, capsules, granules and pellets, for providing and/or improving various parameters. Typical additional ingredients for use in the present invention include excipients, carriers, fillers, glidants, dispersants, plasticizers, wetting agents, anti-tacking agents, neutralization agents, colorants, pigments, opacifiers, flavours, taste improvement agents such as sweeteners, and the like. It is to be understood that these and other benefit agents can be included in the multiple dosage forms and/or added to the pharmaceutical composition, for example, and preferably, in the gelling composition, separate from the multiple dosage forms. The person skilled in the art of formulating pharmaceutical compositions and the multiple oral dosage forms is readily able to identify specific compounds and substances of the above and other types as well as their combinations and amounts to be used. Further guidance can be found in Remington's Pharmaceutical Sciences, 18th Ed. Mack Printing Company, 1990, in particular pages 1289-1329.

Further subject matter of the invention is a pharmaceutical article comprising multiple dosage forms as defined herein and a gelling composition comprising at least one gelling agent as defined herein, wherein the multiple dosage forms and the gelling composition are physically separated.

As mentioned above with respect to the inventive pharmaceutical composition, the gelling composition can contain further gelling agents and/or other pharmaceutically acceptable agents such as preferably one or more pH modifiers, whereby preferred examples of pH modifiers for use in the invention are already outlined above.

In other embodiments of the invention, the pharmaceutical article is comprised of a pharmaceutical composition as defined in claim 1 and, physically separated therefrom, of water or an aqueous solution. Preferably, the physical separation is affected by providing the pharmaceutical composition (without water/aqueous solution) in one container, and the water or aqueous solution in a further, second, container. For use, the water or aqueous solution is combined with the pharmaceutical composition in one of the two containers, or in a different container.

In preferred embodiments, the pharmaceutical article further comprises water and/or an aqueous solution physically separated from the multiple dosage forms and from the at least one gelling agent or gelling composition, respectively.

The "physical separation" according to the invention is any means whereby the multiple dosage forms and the one or more gelling agents or the gelling composition, respectively, are separated from each other such that they do not come into contact, typically until used by the subject for which the pharmaceutical article is envisaged. For example, the multiple dosage forms and the gelling agent(s)(gelling composition can be provided in two separate containers which together make up the pharmaceutical article, optionally, and preferably, in combination with a suitable package and, more preferably, further together with a pharmaceutical leaflet containing instructions for use. The article can also comprise water or an aqueous solution which is also physically separated from the multiple dosage forms and the gelling agent(s)/gelling composition. Preferably, the water or aqueous solution, respectively, is provided in a third container.

In other preferred embodiments of the inventive pharmaceutical article the multiple dosage forms and the gelling composition and, optionally, the water and/or aqueous solution, are provided in a single container comprising at least one compartment containing the multiple dosage form, at least one compartment containing the gelling composition and, optionally, at least one compartment containing the water or aqueous solution, wherein said compartments are separated by an at least partially removable, preferably breakable, physical separation.

Removable physical separations, preferably partially removable physical separations, for use in the pharmaceutical article are known to the skilled person. In one preferred embodiment, the separation is provided by a breakable foil, preferably between each compartment, which foil may preferably embodied as a blister foil typically used for pharmaceuticals. In a preferred embodiment, containing multiple dosage forms, gelling composition, and water or aqueous solution, the compartments are provided in the container such that a first lowest compartment contains the liquid medium, and the two upper compartments contain the multiple dosage forms. The user may then press the contents of the uppermost compartment through the foil such that multiple dosage forms and gelling composition are first mixed, and then by further pressure application, are pressed through the breakable separation, preferably a blister foil, into the lower liquid medium compartment such that the final pharmaceutical composition is reconstituted, typically as a gel or gel-like suspension of the multiple oral dosage forms, and can be administered orally to the subject in need of the treatment preferably by self-administration.

In other preferred embodiments, the pharmaceutical article contains a pharmaceutical composition of the invention (without water or aqueous solution) and water or aqueous solution, respectively, in a single container having two compartments, whereby the pharmaceutical composition is provided in one and the water or aqueous solution, respectively, is provided in a second compartment, and the two compartments are separated by an at least partially removable, preferably, breakable physical separation, more preferred a breakable foil, particularly preferred a blister foil as outlined above for the three-compartment embodiment. For reconstitution, the user, preferably a subject in need of treatment, will at least partially remove the separation between the compartments, preferably by pressing the pharmaceutical composition (without water/aqueous solution) through a blister foil into the compartment containing the liquid medium, preferably water or an aqueous solution.

As regards the pharmaceutical articles as described herein, it is preferred that a container or compartment comprising the multiple dosage forms contains a unit dose of the multiple dosage forms for one administration to the subject. Preferred unit dosages, in particular numbers of multiple dosage forms, are as outlined above.

The present disclosure (not claimed) also relates to methods of preparing pharmaceutical compositions as defined and described herein. In one embodiment, the method for preparing a pharmaceutical composition comprises combining multiple dosage forms as described above, preferably having sizes and/or numbers as outline before, with at least one gelling agent or a gelling composition respectively. The method preferably comprises the further step of combining the resulting mixture of multiple dosage forms and at least one gelling agent or gelling composition, respectively, with a liquid medium, preferably water or an aqueous solution. In a different aspect, there is also provided a method for producing a pharmaceutical composition comprising combining a composition comprising multiple dosage forms as described above and at least one gelling agent or a gelling composition, respectively, with a liquid medium, preferably water or an aqueous solution. As will be understood by the skilled person, the result of this method is typically a gel or gel-like formulation of the multiple dosage forms.

According to the invention, the water for providing a mixture of multiple dosage forms and gelling composition in a liquid medium may be tap water or sterilized water. Aqueous solutions for that purpose are preferably buffered or unbuffered saline solution such as physiological sodium chloride solutions, Ringer or Ringer lactate. The liquid medium may also be selected from other embodiments such as flavoured and/or pigmented or dyed aqueous solutions or even suspensions such as juices, e.g. fruit or vegetable juices, as long as such liquids do not interfere with the formation of a gel or gel-like formulation containing the multiple dosage forms.

The pharmaceutical compositions and/or pharmaceutical articles of the invention as defined in the claims are particular useful in the prevention and/or treatment in a subject suffering from conditions and/or disorders and/or diseases associated with disturbed enterokine release by enteroendocrine cells and/or conditions and/or disorders and/or diseases amenable to increased enterokine release by enteroendocrine cells.

Preferred conditions, disorders or diseases to which the pharmaceutical compositions and/or pharmaceutical articles of the invention can be applied for treatment and/or prevention are metabolic disorders, vascular disorders, neurodegenerative diseases, skeletal diseases and gastroenterological disorders.

Preferred metabolic disorders are selected from insulin resistance, type 2 diabetes mellitus, non-alcoholic fatty liver disease, non-alcoholic steatohepatosis, metabolic syndrome, hyperlipidemia and obesity.

Especially in the context of diabetes, in particular T2DM, and prediabetic conditions, preferably insulin resistance and/or obesity, the therapeutic and/or preventive regimen of the invention has tremendous benefits, since it is known that increased enterokine levels, in particular GLP-1, exerted by the administration of inventive pharmaceutical composition have a beneficial effect of increasing the Adiponectin/Leptin ratio (cf., for example, Unamuno et al. (2019) Nutrients, 11, 2069-2079) which diminishes known inflammation of adipose tissues frequently encountered in patients suffering from T2DM and/or pre-diabetic conditions such as insulin resistance and obesity.

In contrast to prior art applications of enterokine stimulating compositions, the range of indications for which the inventive compositions and/or articles, respectively, can be applied to successfully is broadened tremendously according to the invention, since the inventors have detected and recognized that enterokines released by enterendocrine cells, in particular GLP-1 released by L cells, have various effector cites in the organism, in particular humans, which is particularly displayed by the expression profiles of enterokine receptors, in particular GLP-1 receptors.

For example, GLP-1 shows effects on vascular tissues, in particular a vasodilatory and cardioprotective function (see, e.g. Ban et al. (2008) Circulation 117, 2340-2350) such that the pharmaceutical compositions and/or pharmaceutical articles of the invention can preferably applied for treatment and/or prevention of vascular disorders such as preferably microvascular dysfunction, cardiovascular diseases, cerebrovascular diseases and pulmonary vascular diseases.

Pulmonary diseases are a preferred target for therapy and/or prevention by the inventive embodiments, since expression of GLP-1 receptors is highest in lungs. As preferred classes of pulmonary vascular diseases, pulmonary diseases associated with pneumonia are contemplated, more preferably in pneumonia associated with or caused by, respectively, viral infection.

Since severe forms of coronavirus infection, in particular by coronaviruses causing respiratory pneumoniae caused by or associated with, respectively, infections with SARS-COV-1, SARS-COV-2 and/or MERS, are associated with severe damage of pulmonary vessels, it is proposed by the invention that induction of enterokines, in particular GLP-1, by administration, in particular oral administration, of the pharmaceutical composition, e.g. by use of a pharmaceutical articles as disclosed herein. Particularly preferred therapeutic target of the invention is SARS-COV-2, whereby the administration of the pharmaceutical composition is preferably taken place before the onset of the COVID-19 manifestation, or at least before the occurrence of severe forms comprising development of respiratory conditions such as in particular pneumonia.

In this context, it is to be noted that Glucagon-like-peptide 1 (GLP-1) is best known as an enteroendocrine hormone that orchestrates insulin release in response to ingested nutritional stimuli (Paternoster et al. (2018) Front. Endocrinol. 9, 1-26). GLP-1 has also emerged as an important homeostatic element within the cardiovascular system, where it possesses significant endothelial-protective functions (Helmstädter et al. (2020) Arterioscler. Thromb. Vasc. Biol. 40, 145-158; Sun, Y.-H. et al. (2017) Mol. Med. Rep. 16, 929-936). In the lung, GLP-1 tightens barriers via the upregulation of tight junction proteins in barrier-forming cells ; in alveolar type 2 pneumocytes, GLP-1 stimulates the production of surfactant that, by reducing surface tension, helps minimize fluid accumulation within alveolar spaces (Vara et al. (2001) Am. J. Respir. Crit. Care Med. 163, 840-846). In endothelial cells, GLP-1 inhibits TNF Alpha Converting Enzyme (TACE) expression and activity (Ku et al. (2014) Pharmacol. Res. 84, 18-25): It therefore directly opposes key mechanisms that SARS-CoV-2 commandeers to augment inflammation and compromise barrier function. Accordingly, GLP-1 signalling attenuates TACE-dependent Endothelial Protein C Receptor (EPCR) shedding (Ku et al. (2014), supra); GLP-1 signalling also increases deficient Angiotensin Converting Enzyme 2 (ACE2) levels in pathological settings (Romaní-Pérez. et al. (2015) Endocrinology 156, 3559-3569), presumably by reduced ACE2 shedding. Consistent with this antiinflammatory role, GLP-1 receptor agonists possess several desirable actions, including (i) antagonizing NF-κB signalling (Helmstädter et al. (2020), supra), (ii) reducing immune cell adhesion molecule expression on endothelial cell surfaces (e.g., ICAM-1 and VCAM-1) (Helmstädter et al. (2020), supra; Arakawa et al. (2010) Diabetes 59, 1030-1037), (iii) reducing immune cells cytokine production (Arakawa et al. (2010), *supra*) and (iv) attenuating endothelial cell oxidative stress (Ceriello et al. (2013) Diabetes Care 36, 2346-2350). In fact, several of the risk factors associated with severe COVID-19 (e.g., obesity, diabetes) also associate with reduced GLP-1 secretion and circulating GLP-1 levels.

The present invention therefore expands the repertoire of interventions yielding positive therapeutic effects of GLP-1 effects in COVID-19 patients. The high anti-COVID-19 potential of the embodiments of the invention is underscored by previous findings according to which agonists or supraphysiological levels of native GLP-1 have been utilized to elicit desirable effects. However, normal post-prandial levels of endogenous GLP-1 clearly suffice to stimulate nitric oxide production in the forearm, which increases blood flow, microvascular blood volume, and interstitial oxygen uptake in skeletal muscle cells (Chai et al. (2012) Diabetes 61, 888-896); Dong et al. (2013) Am. J. Physiol. - Endocrinol. Metab. 304, E222-E228) .

A preferred skeletal disease for which the embodiments of the invention can be applied to is osteoporosis.

A further therapeutic aspect of the invention is based on the known proliferative and antiapoptotic effect of enterokines as described herein, specifically GLP-1 and GLP-2, on cells of the gastro-intestinal tract, especially of the gastro-intestinal mucosa (see, for example, Sigalet (2012) J. Anim. Sci. 90, 1224-1232; Aw et al. (2017) Asia-Pac. J. Clin. Oncol. 14, 23-31; and Kissow et al. (2012) Cancer Chemother. Pharmacol. 70, 39-48). By administration of the pharmaceutical compositions of the invention, which may be prepared by use of the pharmaceutical articles as disclosed above, it is possible not only to prevent and/treat malabsorption disorders in affected subjects, but also to treat subjects, in particular human subjects, suffering from disorders, diseases and/or conditions of impaired gastro-intestinal function, in particular due to irregular gastro-intestinal growth including reduced intestinal length and impaired/reduced formation of intestinal mucosa and villi, especially, and preferably, in neonatal and infant children, and/or due to disorders of the intestinal mucosa such as mucositis, preferably resulting from chemotherapy, radiotherapy and/or infections, especially in tumor and/or cancer patients. This treatment aspect of the invention also comprises the prevention of the above gastro-intestinal disorders and diseases.

As disclosed above, the pharmaceutical composition, in particular pharmaceutical gel or gel-like formulations of the multiple dosage forms as described herein, are for use in patients suffering from metabolic disorders, in particular diseases involving aberrant energy household such as diabetes and pre-diabetic conditions as outlined above. In particular in such patients, uptake and transport of conventional oral formulations such as disclosed in the prior art (in particular tablets or capsules containing substantial amounts of active compounds such as glucose) is frequently impaired and/or slowed down considerably under conditions such as gastroparesis (delayed gastric emptying), in particular in patients having infections by *Heliobacter pilori,* neuromuscular dysfunction, and advanced age, and diabetes, in particular T2DM, where in up to 50% of diabetic patients the dysfunction of autonomic nerves, specifically vagal efferent nerves that govern gastric and small intestine peristalsis, is a comorbidity. Therefore, the pharmaceutical compositions and articles of the invention are particularly useful for treatment of subjects, in particular humans, in which one or more of the indications as disclosed herein is/are associated with one or more of the described comorbidities.

Accordingly, the pharmaceutical compositions and/or pharmaceutical articles are particularly useful in cases wherein the above conditions, disorders or diseases are accompanied by at least one condition selected from the group consisting of swallowing difficulty, dysphagia, achalasia, impaired esophageal peristalsis, gastroparesis and impaired intestinal peristalsis.

The present invention discloses a method of treatment of the above-described conditions, disorders and/or diseases comprising the oral administration of an effective amount of a pharmaceutical composition of the invention, in particular the inventive formulation of the multiple dosage forms in a gel or gel-like suspension, to a, preferably human, subject in need thereof, wherein the administration pharmaceutical composition preferably leads to the release of GLP-1 and/or GLP-2, in particular a substantial increase of GLP-1 and/or GLP-2 levels, in said subject. The disclosed method of treatment does not form part of the invention.

In certain embodiments, it is also preferred that the pharmaceutical composition, before administration to the subject, preferably by self administration is reconstituted, preferably by use of the pharmaceutical articles as disclosed herein, by combining a pharmaceutical composition comprising multiple dosage forms as outlined herein and at least one gelling agent or a gelling composition, respectively, as disclosed above, and a suitable liquid as described herein.

In general, the administration of the pharmaceutical composition of the invention leads to a substantial increase of the addressed at least one enterokine, preferably GLP-1, GLP-2, GIP, PYY, CCK and/or neurotensin, particularly preferred GLP-1 and/or GLP-2 and/or PYY, above the respective base-line level of the enterokine, in particular the level of the enterokine in the blood serum/plasma of the subject being treated. In preferred embodiments of the invention, the level of the respective enterokine in the subject's blood serum/plasma increases by at least about 50 % or more, preferably about 50 % to about 200 % or even more such as about 300 %, as compared to the level in the blood plasma/serum of the subject before administration of the pharmaceutical composition of the invention. The increase in enterokine concentration in the subject's blood plasma/serum typically occurs within about 2 to about 6 hours post administration of the composition of the invention, and lasts for a time period of typically about 1 to about 5 hours, preferably about 2 to about 4 hours, most preferred from about 3 to about 4 hours.

According to the invention, the term "effective amount of the pharmaceutical composition" depends on various factors such as the specific condition, disorder or disease to be treated and/or prevented, the age and sex of the subject as well as the general condition of the subject. Furthermore, the "effective amount of the pharmaceutical composition" depends on the type of active compound(s) used. Typically, however, pharmaceutical oral compositions of the invention are preferably administered once daily in one or more unit dosages, more preferably in the fasted state of the subject, particularly preferred at least about 30 min, more preferred at least about 45 min, even more preferred at least 1 hour before the first meal of the day. In preferred embodiments of therapeutic regimens of the invention, one or more unit doses of a pharmaceutical composition as defined herein are administered orally at a time point synchronized with the circadian rhythm of the enteroendokine which release is to be increased by the pharmaceutical composition of the invention. For such circadian synchronization of the administration of the pharmaceutical compositions of the invention, it is preferred that one more unit doses of the pharmaceutical composition are administered in a time period of from about 2 to about 4 hours before the respective enterokine, in particular GLP-1, reaches its circadian maximum in the serum or blood, respectively. In the case of GLP-1 as a particularly preferred enterokine for the purposes of the invention, the serum or blood, respectively, level of this enterokine shows a maximum at or around 11 hrs am. In preferred embodiments of the invention, the one or more unit dosages, particularly for exerting optimal GLP-1 release, are therefore administered at a time point from about 7 hrs am to about 9 hrs am. In the context of the time points given herein as hrs, it is to be understood that these time points refer to the local time zone where the pharmaceutical composition of the invention is to be administered.

With glucose as a preferred active compound, the effective amount is preferably a daily dose of about 0.5 to about 20 g glucose orally administered in one or more unit doses, preferably in the fasted state of the subject as outlined before. A dose of about 1 to about 15 g once daily is more preferred, and about 5 g to about 12 g, most preferred about 10 g glucose once daily may be given as a particularly preferred regimen. The above amounts of the active ingredient, preferably glucose, may be administered in one or more-unit doses of the pharmaceutical compositions, which preferred unit doses, in particular as regards the multiple dosage forms, are already outlined above. A typical amount of glucose contained unit dose of the pharmaceutical composition of the invention is in the range of from about 0.5 g to about 30 g glucose, preferably from about 5 g to about 20 g glucose, more preferably from about 7 g to 15 g glucose, such as ca. 10 g glucose per unit dose of the composition.

As regards the pharmaceutical composition of the invention and its use according to the invention as described above, it is of particular benefit when the composition is formulated, in particular with respect to the sizes, amounts and forms of the multiple dosage forms, such that the at least one compound stimulating enteroendocrine cells to release an enterokine stimulates said cells present in the intestine of the subject from the jejunum to the ileo-cecal valve of the, preferably human, subject.

The therapeutic uses of the invention as defined in the claims also entail a combination of different pharmaceutical compositions wherein the individual composition contain multiple dosage forms exhibiting different travelling profiles in the subject's gastro-intestinal tract and/or contain different compounds stimulating enteroendocrine cells to release at least one enterokine. For example, one composition contains multiple dosage forms comprising glucose and a further composition contains multiple dosage forms comprising an anthocyanine (further combinations and specific examples of active compounds and preferred combinations have already been elaborated above). It is also contemplated that a single pharmaceutical composition contains multiple dosage forms comprising different active compounds as outlined above. A combination of different multiple dosage forms using different active compounds is specifically of benefit when the different compounds are not easily compatible (e.g. as regards their chemical properties) for inclusion in a single dosage form.

In other embodiments of this combinatorial approach, the medical use of the invention comprises administering a first pharmaceutical composition comprising multiple dosage forms as defined herein having a small size as described herein, and administering a second pharmaceutical composition comprising one or more oral dosage forms as otherwise defined herein, but having a size of 3 mm or more, preferably 3 to 10 mm, based on the largest dimension of said first dosage form, wherein the active compound in said first and said second compositions stimulating enteroendocrine cells to release at least one enterokine may be the same or different.

It is, of course, also possible to administer or use, respectively, more than two different sizes and/or more than two different active compounds.

For preparing the multiple dosage forms as described herein, a production method typically comprising the steps of:
(a) preparing a mixture comprising at least one compound stimulating enteroendocrine cells to release at least one enterokine, preferably in combination with at least one disintegrant as defined above,
(b) compressing the mixture obtained in step (a); and
(c) applying to the compressed mixture at least one enteric coating, preferably comprising at least one pH sensitive polymer being preferably selected such that the coating substantially dissolves and/or is substantially degraded in the jejunum of a subject,
wherein the compression and application steps (a) and (b) respectively are selected such that multiple dosage forms having a size as disclosed herein are obtained.

Optionally, the mixture of step (a) and/or the at least one coating of step (c) may comprise further ingredients as outlined above for the pharmaceutical composition *per se.*

Preferred embodiments of the constituents as defined in steps (a) and (c) have already been described in detail above.

According to a further preferred embodiment of the preparation method, more than one coating is applied to the compressed mixture obtained in step (b), i.e. the core component of the pharmaceutical oral dosage form of the invention.

In particularly preferred embodiments of the preparation method, a combination of at least two coatings is applied in step (c). Preferably, a sub-coating of one pH sensitive polymer is applied as a first layer and a coating of a second pH sensitive polymer is applied on the sub-coating as a second layer. For example, the pH sensitive coating can comprise a sub-coating of or comprising, respectively, a hydroxypropylmethyl cellulose as a first layer, onto which a second coating comprising or made of an anionic copolymer of methacrylic acid and methacrylmethacrylate as a second layer. In a further preferred embodiment, a first layer (sub-coating) comprising or made of an anionic polymer of methacrylic acid and methacrylmethacrylate such as an Eudragit^{®}, more preferably Eudragit^{®} FS30D, is applied to the core obtained in step (b) and a second layer comprising or made of a hydroxypropylmethyl cellulose such as AQOAT^{®}, more preferably AQOAT^{®}-HF, is applied onto the first layer. More preferably, the anionic copolymer of methacrylic acid and methacrylmethacrylate, e.g. an Eudragit^{®}, preferably Eurdragit^{®} FS30D, is applied in less amount than the the hydroxypropylmethyl cellulose such as AQOAT^{®}, more preferably AQOAT^{®}-HF, of the second layer. In other words, the thickness of the first layer of this type of combination is lower than the thickness of the second layer in this combination. More specifically, the ratio of amount or thickness, respectively, between first layer and second layer typically ranges from about 1:10 to about 1:50, particularly preferred from about 1:20 to about 1:30. In preferred embodiments of the invention, the one or more coatings are applied by spray-coating in step (c).

For preparing the pharmaceutical composition, the multiple dosage forms are then admixed with a gelling composition, preferably containing further ingredients such as those as outlined above, such as preferably one or more formulation aids, preferably selected from pH modifiers, flavors, taste improvers, lubricants and pigments.

The Figure shows:
- Fig. 1: shows a graphical representation of results of time-dependent GLP-1 blood level measurements (pmol/l) in subjects (n= 20) from 1 hour before to 10 hours after oral administration of a composition according to the invention. Data shown are means +/-SEM.

The following non-limiting example further illustrates the invention:

### EXAMPLE

An exemplary pharmaceutical composition was prepared by mixing the following components (a) and (b):
(a) Multiple dosage forms: Eudragit^{®}-coated beads of ca. 1 mm diameter having a core containing 40 to 70 % (w/w) glucose and a disintegrant in a carrier substance Total weight of glucose in a unit dose of the composition: 10 g. Number of coated beads ca. 20000 to 30000.
(b) Gelling composition:
   Gelling agent mixture (Polysaccharide, modified cellulose, PEG) (84 % (w/w) based on the total weight of the gelling composition)
   pH modifier
   Flavors
   Lubricant
   Pigments

Before oral administration, the pharmaceutical composition was mixed with 25 ml of water per unit dose.

The reconstituted composition was administered orally to 20 subjects. Blood GLP-1 values were measured at the following time points (in relation to time point of oral administration = 0 h): -1.0 h, -0.5 h, 0 h, 0.5 h, 1.0 h, 1.5 h, 2.0 h, 2.5 h, 3.0 h, 3.5 h, 4.0 h, 4.5 h, 5.0 h, 5.5 h, 8.0 h, and 10.0 h

The results are shown in Fig. 1. The pharmaceutical composition exerts a remarkably reproducible (low intra patient and inter-patient variability) and sharp rise in GLP-1 blood concentration (maximum at 2.88 +/- 0.46 fold increase with respect to base line GLP-1 blood level at 1.5 h post administration) as compared to prior art approaches, and the GLP-1 increase over base line value at 1.5 h lasts about 4 h. The sharp rise in GLP-1 blood level starting after 1.5 hours post administration is consistent with a burst release of the active compound (here: glucose) in the terminal jejunum of the subjects as demonstrated by the following data as obtained by Evans et al. (1988) Gut 29, 1035-1041).

**Tab.1: Localisation of pH sensitive telemetry capsule in the gastro-intestinal tract (GI) in dependency of time after intake**

| **GI region** | **Time (hours) after intake** |
|---|---|
| Duodenum | 0.75 to 1.8 |
| Jejunum | 1.8 to 3.8 |
| Ileum | 3.8 to 5.4 |
| Colon | ≥ 5.4 |

Applying the data of Evans et al. (1988), the burst release of the components of the multiple dosage forms contained in the composition of the invention occurred in the (terminal) jejunum of the subjects at a pH environment of > pH 7.0, and in particular ca. 7.3.

## Claims

1. A pharmaceutical composition comprising multiple dosage forms each comprising a core and an enteric coating, wherein the core comprises at least one nutrient compound selected from the group consisting of carbohydrates, fatty acids, bile acids, peptides, amino acids, alcohol amides, and anthocyanins, wherein the size of the dosage forms, with respect to the largest dimension of the dosage forms, is below 3 mm providing for entry of the dosage forms into the intestine of a subject independent of gastric emptying mechanisms, wherein the enteric coating comprises a pH sensitive polymer which substantially degrades and/or dissolves at a pH value being selected such that the coating substantially dissolves and/or is substantially degraded in the terminal jejunum of a subject such that the core is released into the terminal jejunum of the subject, and wherein the composition further comprises a gelling composition containing one or more gelling agents, and wherein the multiple dosage forms and the gelling composition form a heterogenous mixture.

2. The composition of claim 1 wherein the size of each of the dosage forms, with respect to the largest dimension of the dosage forms, is from 0,6 mm to 2,6 mm, preferably from 0,6 mm to 1,7 mm, more preferably from 0,8 mm to 1,2 mm.

3. The composition of claim 2 containing 10000 to 40000 dosage forms, preferably 20000 to 30000 dosage forms.

4. The composition according to any one of the preceding claims wherein the pH sensitive polymer substantially degrades and/or dissolves at a pH value of 5.5 to 7.5, preferably 7.2 to 7.3.

5. The composition according to any one of the preceding claims wherein the pH sensitive polymer is selected from the group consisting of hydroxypropylmethyl celluloses and anionic copolymers of methacrylic acid and methacrylmethacrylate.

6. The composition according to any one of the preceding claims wherein the core contains glucose, and optionally one or more compounds selected from sucralose, fatty acids having 2 to 6 carbon atoms, oleic acid, bile acids, peptides, amino acids, ethanolamides and anthocyanins.

7. The composition of claim 6 wherein the multiple dosage forms each have a glucose content of form 5 % (w/w) to 95 % (w/w). (w/w), preferably from 25 % (w/w) to 75 % (w/w), more preferably from 40 % (w/w) to 70 % (w/w).

8. The composition of claim 6 or 7 containing 0.5 g to 30 g glucose, preferably 5 g to 20 g glucose, more preferably 7 g to 15 g glucose, per unit dose of the composition.

9. The composition according to any one of the preceding claims wherein the multiple dosage forms further contain a substance enhancing enterokine release by the enteroendocrine cells, said substance being selected from GLP-1 degradation inhibitors, substances enhancing L cell maturation, inhibitors of NOTCH signalling, stimulating agents and combinations thereof.

10. The composition of claim 9 wherein the enteroendocrine cell maturation agent is a human milk oligosaccharide (HMO).

11. The composition of claim 9 wherein the inhibitor of GLP1-decgradation inhibitor is DPP-4.

12. The composition of claim 9 wherein the inhibitor of NOTCH signalling is selected from γ-secretase inhibitors.

13. The composition of claim 12 wherein the γ-secretase inhibitor is debenzazepine.

14. The composition of claim 9 wherein the stimulating agent is caffeine.

15. The composition according to any one of the preceding claims wherein the core contains at least one disintegrant providing a burst release of the ingredients of the core from the dosage form upon at least partial dissolving and/or degradation of the enteric coating.

16. The composition according to any one of the preceding claims wherein the multiple dosage forms and the gelling composition are present in a liquid medium, preferably water or an aqueous solution.

17. The composition according to any one of the preceding claims further comprising one or more pH modifiers, preferably contained in the gelling composition.

18. A pharmaceutical article comprising multiple dosage forms as defined according to any one of claims 1 to 15 and a gelling composition comprising at least one gelling agent and, optionally, one or more pH modifiers, wherein the multiple dosage forms and the gelling composition are physically separated.

19. The article of claim 18 further comprising a liquid medium physically separated from the multiple dosage forms and from the gelling composition.

20. The article of claim 18 or 19 wherein the multiple dosage forms and the gelling composition and, optionally, the liquid medium, are provided in a single container comprising at least one compartment containing the multiple dosage form, at least one compartment containing the gelling composition and, optionally, at least one compartment containing the water or aqueous solution, wherein said compartments are separated by a breakable physical separation.

21. A pharmaceutical article comprising the composition according to any one of claims 1 to 17 and comprising a liquid medium physically separated from said composition.

22. The article of claim 21 wherein the composition and the liquid medium are provided in a single container comprising at least one compartment containing the composition and at least one compartment containing the liquid medium, wherein said compartments are separated by a breakable physical separation.

23. The article according to any one of claims 19 to 22 wherein the liquid medium is water or an aqueous solution.

24. The composition according to any one of claims 1 to 17 or the article according to any one of claims 18 to 23 for use in the prevention and/or treatment in a subject suffering from conditions and/or disorders and/or diseases associated with disturbed enterokine release by enteroendocrine cells and/or conditions and/or disorders and/or diseases amenable to increased enterokine release by enteroendocrine cells.

25. The composition or article for use of claim 24 wherein the condition, disorder or disease is selected from metabolic disorders, vascular disorders, neurodegenerative diseases, skeletal diseases and gastroenterologic disorders.

26. The composition or article for use of claim 25 wherein the metabolic disorder is selected from the group consisting of insulin resistance, type 2 diabetes mellitus, non-alcoholic fatty liver disease, non-alcoholic steatohepatosis, metabolic syndrome, hyperlipidemia and obesity; and/or
wherein the vascular disorder is selected from microvascular dysfunction, cardiovascular diseases, cerebrovascular diseases and pulmonary vascular diseases; and/or
wherein the skeletal disease is osteoporosis, and/or
wherein the gastroenterological disorder is selected from the group consisting of impaired gastro-intestinal function and malabsorption conditions.

27. The composition or article for use of claim 26 wherein the metabolic disorder is associated with adipose tissue inflammation.

28. The composition or article for use of claim 26 wherein the pulmonary disease is associated with pneumonia.

29. The composition or article for use of claim 28 wherein the pneumonia is caused by or associated with a viral infection., preferably an infection by a coronavirus causing a respiratory condition, more preferably the viral infection is an infection by a coronavirus selected from the group consisting of SARS-COV-1, SARS-COV-2 and MERS.

30. The composition or article for use according to any one of claims 24 to 29 wherein said condition, disorder or disease is accompanied by at least one condition selected from the group consisting of swallowing difficulty, impaired esophageal peristalsis, gastroparesis and impaired intestinal peristalsis.

## Patentansprüche

1. Pharmazeutische Zusammensetzung, die mehrere Darreichungsformen umfasst, die jeweils einen Kern und eine magensaftresistente Beschichtung umfassen, wobei der Kern mindestens eine Nährstoffverbindung umfasst, die aus der Gruppe, bestehend aus Kohlenhydraten, Fettsäuren, Gallensäuren, Peptiden, Aminosäuren, Alkoholamiden und Anthocyanen, ausgewählt ist, wobei die Größe der Darreichungsformen, bezogen auf die größte Ausdehnung der Darreichungsformen, unter 3 mm beträgt, was den Eintritt der Darreichungsformen in den Darm eines Patienten unabhängig von Magenentleerungsmechanismen ermöglicht, wobei die magensaftresistente Beschichtung ein pH-empfindliches Polymer umfasst, das sich bei einem pH-Wert, der so gewählt ist, dass sich die Beschichtung im terminalen Jejunum eines Subjekts im Wesentlichen auflöst und/oder im Wesentlichen abgebaut wird, so dass der Kern in das terminale Jejunum des Subjekts freigesetzt wird, und wobei die Zusammensetzung außerdem eine Gelzusammensetzung umfasst, die ein oder mehrere Gelmittel enthält, und wobei die mehreren Darreichungsformen und die Gelzusammensetzung ein heterogenes Gemisch bilden.

2. Zusammensetzung nach Anspruch 1, wobei die Größe jeder der Dosierungsformen, bezogen auf die größte Ausdehnung der Dosierungsformen, 0,6 mm bis 2,6 mm, vorzugsweise 0,6 mm bis 1,7 mm, mehr bevorzugt 0,8 mm bis 1,2 mm, beträgt.

3. Zusammensetzung nach Anspruch 2, die 10000 bis 40000 Dosierungsformen, vorzugsweise 20000 bis 30000 Dosierungsformen enthält.

4. Zusammensetzung nach einem der vorhergehenden Ansprüche, wobei das pH-sensitive Polymer bei einem pH-Wert von 5,5 bis 7,5, vorzugsweise 7,2 bis 7,3, im Wesentlichen abgebaut wird und/oder sich auflöst.

5. Zusammensetzung nach einem der vorhergehenden Ansprüche, wobei das pHempfindliche Polymer aus der Gruppe, bestehend aus Hydroxypropylmethylcellulosen und anionischen Copolymeren von Methacrylsäure und Methacrylmethacrylat, ausgewählt ist.

6. Zusammensetzung nach einem der vorhergehenden Ansprüche, wobei der Kern Glucose und gegebenenfalls eine oder mehrere Verbindungen, ausgewählt aus Sucralose, Fettsäuren mit 2 bis 6 Kohlenstoffatomen, Ölsäure, Gallensäuren, Peptiden, Aminosäuren, Ethanolamiden und Anthocyanen, enthält.

7. Zusammensetzung nach Anspruch 6, wobei die mehreren Darreichungsformen jeweils einen Glucosegehalt von 5 % (w/w) bis 95 % (w/w) aufweisen. (w/w), vorzugsweise von 25 % (w/w) bis 75 % (w/w), noch bevorzugter von 40 % (w/w) bis 70 % (w/w).

8. Die Zusammensetzung nach Anspruch 6 oder 7 enthält 0,5 g bis 30 g Glukose, vorzugsweise 5 g bis 20 g Glukose, noch bevorzugter 7 g bis 15 g Glukose, pro Einheitsdosis der Zusammensetzung.

9. Zusammensetzung nach einem der vorhergehenden Ansprüche, wobei die mehreren Darreichungsformen außerdem eine Substanz enthalten, welche die Enterokinfreisetzung durch die enteroendokrinen Zellen verstärkt, wobei die Substanz aus GLP-1-Abbauinhibitoren, Wirkstoffen, welche die L-Zellreifung verstärken, Inhibitoren der NOTCH-Signalkaskade, stimulierenden Mitteln und Kombinationen davon ausgewählt ist.

10. Zusammensetzung nach Anspruch 9, wobei der Wirkstoff zur Reifung der enteroendokrinen Zellen ein Humanmilch-Oligosaccharid (HMO) ist.

11. Zusammensetzung nach Anspruch 9, wobei der Inhibitor des GLP1-Abbauinhibitors DPP-4 ist.

12. Zusammensetzung nach Anspruch 9, wobei der Inhibitor der NOTCH-Signalkaskade aus γ-Sekretase-Inhibitoren ausgewählt ist.

13. Zusammensetzung nach Anspruch 12, wobei der γ-Sekretase-Inhibitor Debenzazepin ist.

14. Die Zusammensetzung nach Anspruch 9, wobei das stimulierende Mittel Koffein ist.

15. Zusammensetzung nach einem der vorhergehenden Ansprüche, wobei der Kern mindestens ein Sprengmittel enthält, das bei mindestens teilweiser Auflösung und/oder Abbau der magensaftresistenten Beschichtung eine zersprengende Freisetzung der Bestandteile des Kerns aus der Darreichungsform bewirkt.

16. Zusammensetzung nach einem der vorhergehenden Ansprüche, wobei die mehreren Darreichungsformen und die Gelzusammensetzung in einem flüssigen Medium, vorzugsweise Wasser oder einer wässrigen Lösung, vorliegen.

17. Zusammensetzung nach einem der vorangehenden Ansprüche, die außerdem einen oder mehrere pH-Modulatoren enthält, die vorzugsweise in der Gelzusammensetzung enthalten sind.

18. Pharmazeutischer Artikel, umfassend mehrere Darreichungsformen, wie sie in einem der Ansprüche 1 bis 15 definiert sind, und eine Gelzusammensetzung, die mindestens ein Gelmittel und gegebenenfalls einen oder mehrere pH-Modulatoren umfasst, wobei die Mehrfachdosierungsformen und die Gelzusammensetzung physikalisch getrennt sind.

19. Artikel nach Anspruch 18, der außerdem ein flüssiges Medium, das physikalisch von den Mehrfachdosierungsformen und der Gelzusammensetzung getrennt ist, umfasst.

20. Artikel nach Anspruch 18 oder 19, wobei die mehreren Darreichungsformen und die Gelzusammensetzung und gegebenenfalls das flüssige Medium in einem einzigen Behälter bereitgestellt werden, der mindestens ein Kompartiment, das die mehreren Darreichungsformen enthält, mindestens ein Kompartiment, das die Gelzusammensetzung enthält, und gegebenenfalls mindestens ein Kompartiment umfasst, das das Wasser oder die wässrige Lösung enthält, wobei die Kompartimente durch eine zerbrechliche physikalische Trennung getrennt sind.

21. Pharmazeutischer Artikel, der die Zusammensetzung nach einem der Ansprüche 1 bis 17 enthält und ein flüssiges Medium umfasst, das physikalisch von der Zusammensetzung getrennt ist.

22. Artikel nach Anspruch 21, wobei die Zusammensetzung und das flüssige Medium in einem einzigen Behälter bereitgestellt werden, der mindestens ein Kompartiment, das die Zusammensetzung enthält, und mindestens ein Kompartiment, welches das flüssige Medium enthält, umfasst, wobei die Kompartimente durch eine zerbrechliche physikalische Trennung getrennt sind.

23. Artikel nach einem der Ansprüche 19 bis 22, wobei das flüssige Medium Wasser oder eine wässrige Lösung ist.

24. Zusammensetzung nach einem der Ansprüche 1 bis 17 oder der Artikel nach einem der Ansprüche 18 bis 23 zur Verwendung in der Vorbeugung und/oder Behandlung eines Subjekts, das an Zuständen und/oder Störungen und/oder Erkrankungen leidet, die mit einer gestörten Enterokinfreisetzung durch enteroendokrine Zellen verbunden sind, und/oder an Zuständen und/oder Störungen und/oder Erkrankungen, die einer erhöhten Enterokinfreisetzung durch enteroendokrine Zellen zugänglich sind.

25. Zusammensetzung oder Artikel zur Verwendung nach Anspruch 24, wobei der Zustand, die Störung oder die Erkrankung aus Stoffwechselstörungen, vaskulären Störungen, neurodegenerativen Erkrankungen, Skeletterkrankungen und gastroenterologischen Störungen ausgewählt ist.

26. Zusammensetzung oder Artikel zur Verwendung nach Anspruch 25, wobei die Stoffwechselstörung aus der Gruppe, bestehend aus Insulinresistenz, Diabetes mellitus Typ 2, nichtalkoholischer Fettlebererkrankung, nichtalkoholischer Steatohepatose, metabolischem Syndrom, Hyperlipidämie und Fettleibigkeit ausgewählt ist,; und/oder
wobei die Gefäßstörung aus mikrovaskulärer Dysfunktion, kardiovaskulären Erkrankungen, zerebrovaskulären Erkrankungen und pulmonalen Gefäßerkrankungen ausgewählt ist; und/oder
wobei die Skeletterkrankung Osteoporose ist, und/oder
wobei die gastroenterologische Störung aus der Gruppe, bestehend aus einer gestörten Magen-Darm-Funktion und Malabsorptionszuständen, ausgewählt ist.

27. Zusammensetzung oder Artikel zur Verwendung nach Anspruch 26, wobei die Stoffwechselstörung mit einer Entzündung des Fettgewebes verbunden ist.

28. Zusammensetzung oder Artikel zur Verwendung nach Anspruch 26, wobei die Lungenerkrankung mit einer Lungenentzündung einhergeht.

29. Zusammensetzung oder Artikel zur Verwendung nach Anspruch 28, wobei die Lungenentzündung durch eine virale Infektion, vorzugsweise eine Infektion durch ein Coronavirus, die einen Atemwegszustand auslöst, verursacht wird wobei die virale Infektion mehr bevorzugt eine Infektion durch ein Coronavirus ist, das aus der Gruppe ausgewählt ist, die aus SARS-COV-1, SARS-COV-2 und MERS besteht.

30. Zusammensetzung oder Artikel zur Verwendung nach einem der Ansprüche 24 bis 29, wobei der Zustand, die Störung oder die Erkrankung von mindestens einem Zustand begleitet wird, der aus der Gruppe ausgewählt ist, die aus Schluckschwierigkeiten, gestörter Ösophagusperistaltik, Gastroparese und gestörter Darmperistaltik besteht.

## Revendications

1. Composition pharmaceutique comprenant des formes de dosage multiples comprenant chacune un noyau et un enrobage entérique, dans laquelle le noyau comprend au moins un composé nutritif choisi dans le groupe constitué de hydrates de carbone, acides gras, acides biliaires, peptides, acides aminés, alcools amides et anthocyanes , dans laquelle la taille des formes de dosage, par rapport à la plus grande dimension des formes de dosage, est inférieure à 3 mm, ce qui permet l'entrée des formes de dosage dans l'intestin d'un sujet, indépendamment des mécanismes de vidange gastrique, dans laquelle l'enrobage entérique comprend un polymère sensible au pH qui se dégrade et/ou se dissout substantiellement à une valeur de pH choisie de telle sorte que l'enrobage se dissout et/ou se dégrade substantiellement dans le jéjunum terminal d'un sujet de telle sorte que le noyau est libéré dans le jéjunum terminal du sujet, et dans laquelle la composition comprend en outre une composition gélifiante contenant un ou plusieurs agents gélifiants, et dans laquelle les formes de dosage multiples et la composition gélifiante forment un mélange hétérogène.

2. Composition selon la revendication 1, dans laquelle la taille de chacune des formes de dosage, par rapport à la plus grande dimension des formes de dosage, est comprise entre 0,6 mm et 2,6 mm, de préférence entre 0,6 mm et 1,7 mm, plus préférentiellement entre 0,8 mm et 1,2 mm.

3. La composition selon la revendication 2 contenant 10000 à 40000 formes de dosage, de préférence 20000 à 30000 formes de dosage.

4. Composition selon l'une quelconque des revendications précédentes, dans laquelle le polymère sensible au pH se dégrade et/ou se dissout essentiellement à un pH compris entre 5,5 et 7,5, de préférence entre 7,2 et 7,3.

5. Composition selon l'une quelconque des revendications précédentes, dans laquelle le polymère sensible au pH est choisi dans le groupe constitué par les hydroxypropylméthylcelluloses et les copolymères anioniques d'acide méthacrylique et de méthacrylméthacrylate.

6. Composition selon l'une quelconque des revendications précédentes, dans laquelle le noyau contient du glucose, et éventuellement un ou plusieurs composés choisis parmi le sucralose, les acides gras ayant de 2 à 6 atomes de carbone, l'acide oléique, les acides biliaires, les peptides, les acides aminés, les éthanolamides et les anthocyanes.

7. La composition selon la revendication 6, dans laquelle les formes de dosage multiples ont chacune une teneur en glucose comprise entre 5 % (p/p) et 95 % (p/p). (p/p), de préférence de 25 % (p/p) à 75 % (p/p), plus préférentiellement de 40 % (p/p) à 70 % (p/p).

8. Composition selon la revendication 6 ou 7 contient de 0,5 g à 30 g de glucose, de préférence de 5 g à 20 g de glucose, plus préférentiellement de 7 g à 15 g de glucose, par unité de dose de la composition.

9. Composition selon l'une des revendications précédentes, dans laquelle les formes de dosage multiples contiennent en outre une substance favorisant la libération d'entérokine par les cellules entéroendocrines, ladite substance étant choisie parmi les inhibiteurs de la dégradation du GLP-1, les substances favorisant la maturation des cellules L, les inhibiteurs de la signalisation NOTCH, les agents stimulants et les combinaisons de ces substances.

10. Composition selon la revendication 9, dans laquelle l'agent de maturation des cellules entéroendocrines est oligosaccharide de lait humain (HMO).

11. Composition selon la revendication 9, dans laquelle l'inhibiteur de la dégradation du GLP1 est la DPP-4.

12. Composition selon la revendication 9, dans laquelle l'inhibiteur de la signalisation NOTCH est choisi parmi les inhibiteurs de la γ-sécrétase.

13. Composition selon la revendication 12, dans laquelle l'inhibiteur de la γ-sécrétase est la débenzazépine.

14. Composition selon la revendication 9, dans laquelle l'agent stimulant est la caféine.

15. Composition selon l'une des revendications précédentes, dans laquelle le noyau contient au moins un désintégrant permettant une libération en rafale des ingrédients du noyau à partir de la forme de dosage lors de la dissolution et/ou de la dégradation au moins partielle de l'enrobage entérique.

16. Composition selon l'une quelconque des revendications précédentes, dans laquelle les formes de dosage multiples et la composition gélifiante sont présentes dans un medium liquide, de préférence de l'eau ou une solution aqueuse.

17. La composition selon l'une quelconque des revendications précédentes comprend en outre un ou plusieurs modificateurs de pH, de préférence contenus dans la composition gélifiante.

18. Article pharmaceutique comprenant des formes de dosage multiples telles que définies selon l'une des revendications 1 à 15 et une composition gélifiante comprenant au moins un agent gélifiant et, éventuellement, un ou plusieurs modificateurs de pH, dans lequel les formes de dosage multiples et la composition gélifiante sont physiquement séparées.

19. Article selon la revendication 18 comprend en outre un medium liquide physiquement séparé des formes de dosage multiples et de la composition gélifiante.

20. Article selon la revendication 18 ou 19, dans lequel les formes de dosage multiples et la composition gélifiante et, éventuellement, le medium liquide, sont fournis dans un récipient unique comprenant au moins un compartiment contenant la forme de dosage multiple, au moins un compartiment contenant la composition gélifiante et, éventuellement, au moins un compartiment contenant l'eau ou la solution aqueuse, dans lequel lesdits compartiments sont séparés par une séparation physique cassable.

21. Article pharmaceutique comprenant la composition selon l'une des revendications 1 à 17 et comprenant un medium liquide physiquement séparé de ladite composition.

22. Article selon la revendication 21, dans lequel la composition et le medium liquide sont fournis dans un seul récipient comprenant au moins un compartiment contenant la composition et au moins un compartiment contenant le medium liquide, dans lequel lesdits compartiments sont séparés par une séparation physique cassable.

23. Article selon l'une des revendications 19 à 22, dans lequel le medium liquide est de l'eau ou une solution aqueuse.

24. Composition selon l'une des revendications 1 à 17 ou article selon l'une des revendications 18 à 23 à utiliser dans la prévention et/ou le traitement chez un sujet souffrant d'états et/ou de troubles et/ou de maladies associés à une libération perturbée d'entérocine par les cellules entéroendocrines et/ou d'états et/ou de troubles et/ou de maladies susceptibles d'augmenter la libération d'entérocine par les cellules entéroendocrines.

25. Composition ou article à utiliser selon la revendication 24, dans lequel le trouble ou la maladie est choisi parmi les troubles métaboliques, les troubles vasculaires, les maladies neurodégénératives, les maladies du squelette et les troubles gastro-entérologiques.

26. Composition ou article à utiliser selon la revendication 25, dans lequel le trouble métabolique est choisi dans le groupe constitué par la résistance à l'insuline, le diabète sucré de type 2, la stéatose hépatique non alcoolique, la stéatohépatose non alcoolique, le syndrome métabolique, l'hyperlipidémie et l'obésité ; et/ou
dans lequel le trouble vasculaire est choisi parmi les dysfonctionnements microvasculaires, les maladies cardiovasculaires, les maladies cérébrovasculaires et les maladies vasculaires pulmonaires ; et/ou
dans lequel la maladie du squelette est l'ostéoporose, et/ou
dans lequel le trouble gastro-entérologique est choisi dans le groupe constitué par l'altération de la fonction gastro-intestinale et les conditions de malabsorption.

27. Composition ou article à utiliser selon la revendication 26, dans lequel le trouble métabolique est associé à une inflammation du tissu adipeux.

28. Composition ou article à utiliser selon la revendication 26, dans lequel la maladie pulmonaire est associée à la pneumonie.

29. Composition ou article à utiliser selon la revendication 28, dans lequel la pneumonie est causée par ou associée à une infection virale, de préférence une infection par un coronavirus causant une affection respiratoire, plus préférentiellement l'infection virale est une infection par un coronavirus choisi dans le groupe constitué de SARS-COV-1, SARS-COV-2 et MERS.

30. Composition ou article à utiliser selon l'une des revendications 24 à 29, dans lequel cette condition, ce trouble ou cette maladie s'accompagne d'au moins une condition choisie dans le groupe constitué par les difficultés de déglutition, l'altération du péristaltisme œsophagien, la gastroparésie et l'altération du péristaltisme intestinal.
